# FASCICULE DE BREVET EUROPEEN

(11) **EP 1 437 124 B1**
(45) Date de publication et mention de la délivrance du brevet: **26.03.2008**
(21) Numéro de dépôt: 03292797.2
(22) Date de dépôt: 07.11.2003
(51) Int. Cl.: A61Q 19/00

(54) **Composition pour le traitement d'une peau à tendance acnéique**
Zusamensetzung zur Aknebehandlung
Composition for the treatment of acne

(30) Priorité: 08.01.2003 FR 0300146
(43) Date de publication de la demande: 14.07.2004
(73) Titulaire: L'ORÉAL, 75008 Paris (FR)
(72) Inventeur: Marion, Catherine, 92160 Antony (FR)
(74) Mandataire: Renard, Emmanuelle

(56) Documents cités:
- EP-A- 1 172 103
- WO-A-00/64472
- WO-A-98/48768
- US-A- 6 071 541
- PATENT ABSTRACTS OF JAPAN vol. 2000, no. 26, 1 juillet 2002 (2002-07-01) & JP 2001 261546 A (KANEBO LTD), 26 septembre 2001 (2001-09-26)

## Description

La présente invention se rapporte à une composition renfermant, dans un milieu physiologiquement acceptable, au moins un hydroxyacide, au moins un sel de cuivre, au moins un sel de zinc, au moins un extrait d'algue et au moins un halogénoalkynyl carbamate.

Elle concerne également un procédé de traitement cosmétique d'une peau à tendance acnéique comprenant l'application topique sur ladite peau de la composition précitée.

La peau grasse ou hyperséborrhéique se caractérise par une peau luisante, parfois d'aspect huileux, épaisse, aux pores pilosébacés dilatés. L'hypersécrétion sébacée est le plus souvent liée à une hyperandrogénie dûe soit à une hyperproduction d'androgènes par une glande endocrine, soit à une hyperproduction périphérique au niveau de la glande sébacée à partir des androgènes et/ou proandrogènes environnants.

Cette hypersécrétion sébacée, combinée à une accumulation de cellules épithéliales résultant d'une multiplication cellulaire anormale, peut conduire à une obstruction des follicules sébacés, localisés en particulier sur le visage, qui peut évoluer en comédons.

En outre, une bactérie résidente anaérobie, *Propioni bacterium acnes*, prolifère dans cet environnement riche en sébum et en cellules folliculaires et peut conduire localement à une inflammation.

Une peau grasse à tendance acnéique présente donc généralement des imperfections cutanées, des pores dilatés, une texture inhomogène de la peau et des rougeurs.

Le traitement cosmétique de ce type de peau implique en général l'application topique sur la peau d'un mélange de composés permettant d'agir sur les différentes causes des imperfections et en particulier de contrôler la production de sébum (rétinoïdes, sels de zinc, extraits de cannelle, d'ulmaire ou de laminaire) et/ou de favoriser la desquamation (α- et β-hydroxyacides) et/ou de limiter la prolifération microbienne (iodopropynyl butylcarbamate, triclosan, octoxyglycérine, octanoylglycine).

Toutefois, si la plupart des composés de l'art antérieur permettent effectivement de traiter les imperfections cutanées des peaux grasses, ils ne sont pas suffisants pour favoriser la reconstruction d'un épiderme, en particulier d'une couche cornée, de bonne qualité. Seuls les rétinoïdes pourraient avoir cet effet, en raison de leur capacité à augmenter la prolifération épidermique tout en ralentissant la différentiation terminale des cellules épidermiques. Leur utilisation est toutefois limitée par leur instabilité chimique vis-à-vis de l'oxydation et par leur caractère irritant qui les rend mal adaptés au traitement des peaux sensibles.

Il reste donc nécessaire de disposer de composés permettant de lutter efficacement contre les imperfections cutanées tout en respectant les peaux sensibles.

Or, la Demanderesse a découvert avec étonnement qu'un mélange de différents composés permettait d'atteindre ce but. Ces composés ont, en combinaison, un effet analogue à celui des rétinoïdes sans présenter leurs inconvénients.

L'invention a donc pour objet une composition renfermant, dans un milieu physiologiquement acceptable, au moins un α-hydroxyacide, au moins un sel de cuivre, au moins un sel de zinc, au moins un extrait d'algue et au moins un halogénoalkynyl carbamate.

Elle a également pour objet un procédé de traitement cosmétique d'une peau à tendance acnéique comprenant l'application topique sur ladite peau de la composition précitée.

Les hydroxyacides utilisables selon l'invention peuvent être choisis parmi les α-hydroxyacides et β-hydroxyacides.

Des exemples d'α-hydroxyacides sont les acides glycolique, lactique, malique, tartrique et mandélique, l'acide glycolique étant préféré pour une utilisation dans la présente invention. Des exemples de β-hydroxyacides sont l'acide salicylique, ses esters et sels, et ses dérivés alkylés tels que l'acide n-octanoyl-5-salicylique. Ces composés peuvent représenter de 0,01 à 1% du poids total de la composition.

Comme sels de zinc et de cuivre, on peut choisir indépendamment les sels organiques et inorganiques de ces métaux.

Des exemples de sels organiques comprennent les sels formés à partir d'un contre-anion choisi parmi : un anion citrate, oxalate, acétate, gluconate, lactate, tartrate, maléate, benzoate, propionate, salicylate, ascorbate, formate, succinate, folinate, aspartate, phthalate, oléate, palmitate, stéarate, lauryl sulfate, lanolate, myristate, béhénate, caséinate, cyclamate, pantothénate, polyaminopolycarboxylate, thioglycolate, laurate, ricinoléate, pidolate, sorbate ou glycyrrhizinate.

Des exemples de sels inorganiques comprennent les sels formés à partir d'un contre-anion choisi parmi : un anion nitrate, sulfate, halogénure, carbonate, bicarbonate, hydroxyde, peroxyde, nitrure, sulfure, bisulfate, persulfate, glycérophosphate, hypophosphate ou borate.

Les pidolates de zinc et de cuivre sont préférés pour une utilisation dans la présente invention. Ces composés peuvent représenter chacun de 0,01 à 1% du poids total de la composition.

L'extrait d'algue utilisable selon la présente invention peut notamment être un extrait d'algue verte, en particulier de la souche *Chlamydomonas reinhardtii*, notamment un extrait de fractions cytoplasmiques tel que celui commercialisé par la société RICHTER sous la dénomination commerciale Stimoderm®. Cet extrait, qui est connu pour stimuler les cellules épidermiques, peut être obtenu par culture biotechnologique suivie d'une désintégration douce de la biomasse en vue de récupérer la fraction cytoplasmique, puis d'une élimination des membranes cellulaires. L'extrait de *Chlamydomonas reinhardtii* peut représenter de 0,001 à 0,01% du poids total de la composition.

Comme halogénoalkynyl carbamate, on peut citer en particulier le 3-iodo-2-propynylbutyl carbamate. Ce composé peut avantageusement être utilisé en mélange avec le monococoate de polyéthylène glycol, le dicocoate de polyéthylène glycol et le polyéthylène glycol (4 OE) ; il s'agit plus particulièrement du mélange vendu par la société LONZA sous la dénomination "Glycacil L" où les constituants se trouvent dans un rapport 10/40/40/10 : 10 % de 3-iodo-2-propynylbutyl carbamate, 40 % de monococoate de polyéthylène glycol, 40 % de dicocoate de polyéthylène glycol et 10% de polyéthylène glycol (4 OE).

Le 3-iodo-2-propynylbutyl carbamate peut également être utilisé dans la composition selon l'invention sous forme solide, notamment en mélange avec un carbonate ou bicarbonate de métal alcalin ou de métal alcalino-terreux, et plus particulièrement du bicarbonate de sodium. Un tel mélange est notamment commercialisé par la société LONZA sous la dénomination commerciale "Glycacil S". Il peut en variante être utilisé sous forme stabilisée à 6% dans l'eau, tel que le mélange commercialisé par la société LONZA sous la dénomination commerciale "Glycacil 2000"

La composition selon l'invention renferme de préférence de 0,01 à 0,1% en poids de 3-iodo-2-propynylbutyl carbamate, par rapport au poids total de la composition.

La composition selon l'invention peut se présenter sous toutes les formes galéniques classiquement utilisées pour une application topique et notamment sous forme de gels aqueux, de solutions aqueuses ou hydroalcooliques. Elles peut aussi, par ajout d'une phase grasse ou huileuse, se présenter sous forme de dispersions du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou des dispersions cire/phase aqueuse. Ces compositions sont préparées selon les méthodes usuelles.

La composition utilisée selon l'invention peut être plus ou moins fluide et avoir l'aspect d'une crème blanche ou colorée, d'une pommade, d'un lait, d'une lotion, d'un sérum, d'une pâte, d'une mousse. Elle peut être éventuellement appliquée sur le contour de l'oeil sous forme solide, et par exemple sous forme coulée, en coupelle ou sous forme de stick.

Lorsque la composition est sous forme d'émulsion, la proportion de la phase huileuse de l'émulsion peut aller par exemple de 5 à 80 % en poids, et de préférence de 5 à 50 % en poids par rapport au poids total de la composition. Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques.

Comme matières grasses utilisables dans l'invention, on peut utiliser les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, par exemple; et leurs mélanges.

La composition selon l'invention peut également contenir les adjuvants habituels dans les domaines cosmétique et dermatologique, tels que les gélifiants hydrophiles ou lipophiles, les actifs, les conservateurs, les solvants, les parfums, les charges, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés avantageuses du mélange selon l'invention.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, et la silice hydrophobe.

Comme charges, on peut citer par exemple, les particules de polyamide (Nylon) et notamment celles vendues sous les dénominations ORGASOL par la société Atochem ; les fibres de polamide ; les poudres ou billes de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les microsphères de polyméthacrylate de méthyle, commercialisées sous la dénomination MICROSPHERE M-100 par la société Matsumoto ou sous la dénomination COVABEAD LH85 par la société Wackherr ; les poudres de copolymère éthylène-acrylate, comme celles commercialisées sous la dénomination FLOBEADS par la société Sumitomo Seika Chemicals ; les poudres expansées telles que les microsphères creuses et notamment, les microsphères formées d'un terpolymère de chlorure de vinylidène, d'acrylonitrile et de méthacrylate et commercialisées sous la dénomination EXPANCEL par la société Kemanord Plast sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm), ou les microsphères commercialisées sous la dénomination MICROPEARL F 80 ED par la société Matsumoto ; les poudres de matériaux organiques naturels tels que les poudres d'amidon, notamment d'amidons de maïs, de blé ou de riz, réticulés ou non, telles que les poudres d'amidon réticulé par l'anhydride octénylsuccinate, commercialisées sous la dénomination DRY-FLO par la société National Starch ; les microbilles de résine de silicone telles que celles commercialisées sous la dénomination TOSPEARL par la société Toshiba Silicone, notamment TOSPEARL 240 ; la silice ; les oxydes métalliques tels que le dioxyde de titane ou l'oxyde de zinc ; le mica ; et leurs mélanges.

Il peut également être avantageux d'ajouter à la composition selon l'invention des astringents, tels que l'éthanol ou un extrait aqueux de tiges d'hamamélis sèches; des agents anti-inflammatoires, tels qu'un extrait saccharidique de *Laminaria digitata* ou l'hexapaptide commercialisé par la société VINCIENCE sous la dénomination commerciale Modulene®; et des agents hydratants, tels que la glycérine ou le pidolate d'arginine.

L'invention sera maintenant illustrée par les exemples non limitatifs suivants.

### EXEMPLES

### Exemple 1 : Mise en évidence de l'efficacité du mélange selon l'invention

On a testé sur explants de peau humaine maintenus en survie un mélange constitué de : 0,25% de pidolate de zinc ; 0,2% de Glycacil L ; 5% d'extrait de *Chlamydomonas reinhardtii* à 0,9% dans l'eau ; 0,05% de pidolate de cuivre ; et 1% d'acide glycolique dans un excipient neutre constitué de 85,9% de cyclohexasiloxane ; 7% d'eau ; et 0,6% d'huile de ricin hydrogénée polyéthoxylée (60 OE).

Les explants de peau issus de chirurgie plastique provenaient de trois donneurs différents. Ils ont été déposés dans des inserts eux-mêmes positionnés sur des puits de culture. Un milieu de culture (antibiotiques, sérum de veau foetal) a été ajouté au fond des puits, un passage s'effectuant par diffusion lente entre les deux compartiments par l'intermédiaire d'une membrane poreuse (3 µm).

On a appliqué par léger massage, deux fois par jour pendant 3 jours, les produits à tester, constitués soit du mélange mentionné précédemment, soit d'un excipient constitué exclusivement de cyclohexasiloxane. Des explants de peau non traités et non soumis au corticoïde ont été utilisés comme témoin.

Trois marqueurs ont été observés : la prolifération épithéliale, analysée après immunomarquage à l'aide d'un anticorps anti-Ki67 ; la différenciation terminale, analysée par immunohistochimie de la transglutaminase de type I ; et l'épaisseur de la couche cornée, évaluée par histologie après coloration par l'hémalun-éosine.

Aux fins d'évaluer la modulation de la prolifération, un modèle expérimental a été réalisé pour obtenir une modulation négative du potentiel mitotique de la peau, en ajoutant à J0 aux explants de peau un corticoïde (Solupred®) dans le milieu de culture à la dilution de 0,7 µg/ml.

Les résultats obtenus étaient les suivants :
1. Prolifération épithéliale : le pourcentage de cellules basales en division des peaux sous corticoïdes traitées par le mélange d'actifs était de 20,6%. Ce chiffre était nettement supérieur à celui obtenu pour les peaux traitées par l'excipient (6,7%) et même à celui obtenu pour le témoin (13,9%).
2. Différenciation terminale : le score semi-quantitatif de différenciation observé pour les peaux traitées par le mélange d'actifs était inférieur à celui observé pour le témoin non traité. Les peaux traitées avec l'excipient présentaient un score de différenciation plus élevé que le témoin.
3. Epaisseur de la couche cornée : le score semi-quantitatif observé pour les peaux traitées par le mélange d'actifs était inférieur à celui observé pour les peaux traitées par l'excipient, lui-même inférieur au témoin non traité.

Il résulte de ce qui précède que le mélange d'actifs testé améliore la prolifération cellulaire, diminue la différenciation terminale de l'épiderme et réduit l'épaisseur de la couche cornée, de sorte qu'il présente une activité analogue aux rétinoïdes qui contribue à la reconstruction d'un épiderme plus sain et de meilleure qualité.

### Exemple 2 : Composition cosmétique

La composition ci-dessous est préparée de manière classique pour l'homme du métier. Les quantités sont indiquées en pourcentages pondéraux.

| | |
|---|---|
| Ethanol | 12,50% |
| Pidolate de zinc | 0,25% |
| Pidolate de cuivre | 0,05% |
| Mélange de tensioactifs et iodopropynyl | |
| butylcarbamate (Glycacil L de LONZA) | 0,20 % |
| Extrait de *Chlamydomonas reinhardtii* | |
| à 0,9% dans l'eau (Stimoderm de RICHTER) | 0,50% |
| Acide glycolique | 0,10 % |
| Charges | 13,00% |
| EDTA | 0,05% |
| Gélifiants | 2,00 % |
| Neutralisants | qsp pH 5,6 |
| Eau | qsp 100 % |

Cette composition, appliquée quotidiennement sur une peau à tendance acnéique, permet de résorber ses imperfections, de resserrer les pores et d'homogénéiser la texture de la peau. Elle convient aux peaux sensibles.

## Revendications

1. Composition renfermant, dans un milieu physiologiquement acceptable, au moins un α-hydroxyacide, au moins un sel de cuivre, au moins un sel de zinc, au moins un extrait d'algue et au moins un halogénoalkynyl carbamate.

2. Composition selon la revendication 1, **caractérisée en ce que** ledit hydroxyacide est un α-hydroxyacide choisi parmi les acides glycolique, lactique, malique, tartrique et mandélique.

3. Composition selon la revendication 1, **caractérisée en ce que** ledit hydroxyacide est un β-hydroxyacide choisi parmi l'acide salicylique, ses esters et sels, et ses dérivés alkylés

4. Composition selon la revendication 2 ou 3, **caractérisée en ce que** ledit hydroxyacide représente de 0,01 à 1% du poids total de la composition.

5. Composition selon la revendication 1, **caractérisée en ce que** lesdits sels de cuivre et de zinc sont indépendamment choisis parmi les sels organiques et inorganiques.

6. Composition selon la revendication 5, **caractérisée en ce que** les sels organiques sont formés à partir d'un contre-anion choisi parmi : un anion citrate, oxalate, acétate, gluconate, lactate, tartrate, maléate, benzoate, propionate, salicylate, ascorbate, formate, succinate, folinate, aspartate, phthalate, oléate, palmitate, stéarate, lauryl sulfate, lanolate, myristate, béhénate, caséinate, cyclamate, pantothénate, polyaminopolycarboxylate, thioglycolate, laurate, ricinoléate, pidolate, sorbate ou glycyrrhizinate.

7. Composition selon la revendication 5, **caractérisée en ce que** lesdits sels inorganiques sont formés à partir d'un contre-anion choisi parmi : un anion nitrate, sulfate, halogénure, carbonate, bicarbonate, hydroxyde, peroxyde, nitrure, sulfure, bisulfate, persulfate, glycérophosphate, hypophosphate ou borate.

8. Composition selon l'une quelconque des revendications 1 à 7, **caractérisée en ce que** les sels de zinc et de cuivre sont le pidolate de zinc et le pidolate de cuivre.

9. Composition selon la revendication 8, **caractérisée en ce que** lesdits sels de zinc et de cuivre représentent chacun de 0,01 à 1% du poids total de la composition.

10. Composition selon la revendication 1, **caractérisée en ce que** ledit extrait d'algue est un extrait de *Chlamydomonas reinhardtii.*

11. Composition selon la revendication 10, **caractérisée en ce que** ledit extrait d'algue représente de 0,001 à 0,01% du poids total de la composition.

12. Composition selon la revendication 1, **caractérisée en ce que** ledit halogénoalkynyl carbamate, est le 3-iodo-2-propynylbutyl carbamate.

13. Composition selon la revendication 12, **caractérisée en ce que** ledit halogénoalkynyl carbamate représente de 0,01 à 0,1% du poids total de la composition.

14. Procédé de traitement cosmétique d'une peau à tendance acnéique comprenant l'application topique sur ladite peau de la composition précitée.

## Claims

1. Composition containing, in a physiologically acceptable medium, at least one α-hydroxy acid, at least one copper salt, at least one zinc salt, at least one algal extract and at least one haloalkynyl carbamate.

2. Composition according to Claim 1, **characterized in that** the said hydroxy acid is an α-hydroxy acid chosen from glycolic acid, lactic acid, malic acid, tartaric acid and mandelic acid.

3. Composition according to Claim 1, **characterized in that** the said hydroxy acid is a β-hydroxy acid chosen from salicylic acid, esters and salts thereof, and alkyl derivatives thereof.

4. Composition according to Claim 2 or 3, **characterized in that** the said hydroxy acid represents from 0.01% to 1% of the total weight of the composition.

5. Composition according to Claim 1, **characterized in that** the said copper and zinc salts are chosen independently from organic and mineral salts.

6. Composition according to Claim 5, **characterized in that** the organic salts are formed from a counter-anion chosen from: a citrate, oxalate, acetate, gluconate, lactate, tartrate, maleate, benzoate, propionate, salicylate, ascorbate, formate, succinate, folinate, aspartate, phthalate, oleate, palmitate, stearate, lauryl sulfate, lanolate, myristate, behenate, caseinate, cyclamate, pantothenate, polyaminopolycarboxylate, thioglycolate, laurate, ricinoleate, pidolate, sorbate or glycyrrhizinate anion.

7. Composition according to Claim 5, **characterized in that** the said mineral salts are formed from a counter-anion chosen from: a nitrate, sulfate, halide, carbonate, bicarbonate, hydroxide, peroxide, nitride, sulfide, bisulfate, persulfate, glycerophosphate, hypophosphate or borate anion.

8. Composition according to any one of Claims 1 to 7, **characterized in that** the zinc and copper salts are zinc pidolate and copper pidolate.

9. Composition according to Claim 8, **characterized in that** the said zinc and copper salts each represent from 0.01% to 1% of the total weight of the composition.

10. Composition according to Claim 1, **characterized in that** the said algal extract is an extract of *Chlamydomonas reinhardtii.*

11. Composition according to Claim 10, **characterized in that** the said algal extract represents from 0.001% to 0.01% of the total weight of the composition.

12. Composition according to Claim 1, **characterized in that** the said haloalkynyl carbamate is 3-iodo-2-propynylbutyl carbamate.

13. Composition according to Claim 12, **characterized in that** the said haloalkynyl carbamate represents from 0.01% to 0.1% of the total weight of the composition.

14. Cosmetic process for treating acne-prone skin, comprising the topical application of the abovementioned composition to the said skin.

## Patentansprüche

1. Zusammensetzung, die in einem physiologisch akzeptablen Medium mindestens eine α-Hydroxysäure, mindestens ein Kupfersalz, mindestens ein Zinksalz, mindestens einen Algenextrakt und mindestens ein Halogenoalkinylcarbamat enthält.

2. Zusammensetzung nach Anspruch 1, **dadurch gekennzeichnet, dass** die Hydroxysäure eine α-Hydroxysäure ist, die unter Glycolsäure, Milchsäure, Apfelsäure, Weinsäure und Mandelsäure ausgewählt ist.

3. Zusammensetzung nach Anspruch 1, **dadurch gekennzeichnet, dass** die Hydroxysäure eine β-Hydroxysäure ist, die unter Salicylsäure, ihren Estern und Salzen und ihren Alkylderivaten ausgewählt ist.

4. Zusammensetzung nach Anspruch 2 oder 3, **dadurch gekennzeichnet, dass** die Hydroxysäure 0,01 bis 1 % des Gesamtgewichts der Zusammensetzung ausmacht.

5. Zusammensetzung nach Anspruch 1, **dadurch gekennzeichnet, dass** die Kupfersalze und Zinksalze unabhängig voneinander unter den organischen und anorganischen Salzen ausgewählt sind.

6. Zusammensetzung nach Anspruch 5, **dadurch gekennzeichnet, dass** die organischen Salze mit einem Gegenion gebildet sind, das ausgewählt ist unter: Citrat, Oxalat, Acetat, Gluconat, Lactat, Tartrat, Maleat, Benzoat, Propionat, Salicylat, Ascorbat, Format, Succinat, Folinat, Aspartat, Phthalat, Oleat, Palmitat, Stearat, Laurylsulfat, Lanolat, Myristat, Behenat, Caseinat, Cyclamat, Pantothenat, Polyaminopolycarboxylat, Thioglycolat, Laurat, Ricinoleat, Pidolat, Sorbat oder Glycyrrhizinat.

7. Zusammensetzung nach Anspruch 5, **dadurch gekennzeichnet, dass** die anorganischen Salze mit einem Gegenion gebildet sind, das ausgewählt ist unter: Nitrat, Sulfat, Halogenid, Carbonat, Bicarbonat, Hydroxid, Peroxid, Nitrid, Sulfid, Bisulfat, Persulfat, Glycerophosphat, Hypophosphat oder Borat.

8. Zusammensetzung nach einem der Ansprüche 1 bis 7, **dadurch gekennzeichnet, dass** es sich bei dem Zink- und Kupfersalz um Zinkpidolat und Kupferpidolat handelt.

9. Zusammensetzung nach Anspruch 8, **dadurch gekennzeichnet, dass** das Zink- und Kupfersalz jeweils 0,01 bis 1 % des Gesamtgewichts der Zusammensetzung ausmacht.

10. Zusammensetzung nach Anspruch 1, **dadurch gekennzeichnet, dass** der Algenextrakt ein Extrakt von *Chlamydomonas reinhardtii* ist.

11. Zusammensetzung nach Anspruch 10, **dadurch gekennzeichnet, dass** der Algenextrakt 0,001 bis 0,01 % des Gesamtgewichts der Zusammensetzung ausmacht.

12. Zusammensetzung nach Anspruch 1, **dadurch gekennzeichnet, dass** das Halogenoalkinylcarbamat das 3-Iod-2-propinylbutyl-carbamat ist.

13. Zusammensetzung nach Anspruch 12, **dadurch gekennzeichnet, dass** das Halogenoalkinylcarbamat 0,01 bis 0,1 % des Gesamtgewichts der Zusammensetzung ausmacht.

14. Verfahren zur kosmetischen Behandlung von Haut mit Aknetendenz, das das topische Aufbringen der genannten Zusammensetzung auf die Haut umfasst.
